# EUROPÄISCHE PATENTSCHRIFT

(11) **EP 2 666 418 B1**
(45) Veröffentlichungstag und Bekanntmachung des Hinweises auf die Patenterteilung: **12.07.2017**
(21) Anmeldenummer: 13168533.1
(22) Anmeldetag: 21.05.2013
(51) Int. Cl.: A61B 17/16, A61B 17/17

(54) **Chirurgisches Fräswerkzeug**
Surgical milling tool
Outil de fraisage chirurgical

(30) Priorität: 25.05.2012 DE 102012208816
(43) Veröffentlichungstag der Anmeldung: 27.11.2013
(60) Teilanmeldung: 14154006.2
(73) Patentinhaber: Mathys AG Bettlach, 2544 Bettlach (CH)
(72) Erfinder: Dallmann, Frank, 04626 Schmölln (DE); Baumgartner Marti, Roger, Seaforth, NSW 2092 (AU)
(74) Vertreter: Körfer, Thomas

(56) Entgegenhaltungen:
- WO-A1-2011/012318
- DE-T2- 69 524 703
- DE-T2- 69 606 847
- DE-U1-212006 000 089
- US-A1- 2012 123 419
- US-B2- 8 075 563

## Beschreibung

Die Erfindung betrifft ein modulares, chirurgisches Fräswerkzeug zur Abtragung von Knochen oder Knorpelgewebe, insbesondere zur Herstellung einer Ausnehmung für ein Schultergelenk- und/oder ein Hüftgelenkimplantat.

Degenerierte Hüft- oder Schultergelenke können heutzutage durch künstliche Hüft- bzw. Schulterimplantate ersetzt werden. Zur Aufnahme z. B. einer künstlichen Schulterpfanne, muss in die entsprechende Knochenoberfläche im Schulterblatt eine Ausnehmung eingefräst werden, in die das Implantat eingesetzt wird.

Dabei wird üblicherweise ein Fräswerkzeug verwendet, wie es beispielsweise in der WO 2011/012318 A1 offenbart ist. Der beschriebene Fräser umfasst einen Schaft und einen Fräskopf, der mit dem Schaft gekoppelt ist. Durch Drehung um die Längsachse des Schaftes wird die Bewegung auf den Fräskopf übertragen. Der Fräskopf selbst hat eine längliche Kontur und weist Schneidelemente auf, die sich radial nach außen von der Längsachse zur umfänglichen Wand erstrecken.

Zur Positionierung des Fräsers wird üblicherweise ein Führungsstab im Knochen eingebracht, der die Drehachse des Fräskopfs markiert. Der Fräskopf selbst weist eine zentrale Öffnung entlang der Drehachse auf, in die der Führungsstab eingeführt wird. Der Fräskopf wird dann entlang des Führungsstabs zum Knochen geführt. Da Weichteile, wie Muskeln und Bänder, insbesondere im Schulterbereich, nur wenig beiseite gezogen werden können, ist oftmals nicht die gesamte auszufräsende Fläche direkt und senkrecht zum Führungsstab zugänglich.

Um ein solches Fräswerkzeug auf der Schulterpfanne zu positionieren, müssen der Oberarmknochen und Weichteile mit Hilfe von Knochenhebeln und Retraktoren zur Seite gezogen werden. Dies ist nur sehr beschränkt möglich, sodass oftmals nicht die gesamte Fläche der Schulterpfanne freiliegt.

In der WO 2011/012318 A1 ist der Fräskopf deshalb länglich geformt, sodass der Fräskopf mit der schmaleren Ausdehnung in Richtung der Weichteile angesetzt wird und durch Drehung des Fräskopfes die längere Seite in den durch die Weichteile verdeckten Gelenkspalt eingeführt wird. Somit kann eine Fläche mit dem Radius der längeren Seite des Fräskopfes erzeugt werden.

Dies hat jedoch den Nachteil, dass durch die längliche Kontur des Fräskopfs die Schneiden radial unterschiedlich abgenutzt werden und der Fräskopf mit seiner längeren Seite bei Beginn der Drehbewegung an Haltewerkzeugen zum Weghalten der Weichteile anstößt, die bei der Positionierung nicht erkannt wurden. Dies hat eine unkontrollierte Bewegung der Haltewerkzeuge oder gar eine Verletzung des Gewebes zur Folge. Auch der Fräskopf kann dabei beschädigt werden. Bei sehr geringem Platzverhältnis ist zudem das Positionieren des Fräskopfs nach wie vor schwierig bis unmöglich.

US 2012/0123419 A1 beschreibt ein chirurgisches Fräswerkzeug zur Vorbereitung eines Schultergelenks für die Implantation einer Prothese. Das Werkzeug umfasst eine Fräse und einen Schaft. Die Fräse kann einen kreisförmigen Umfang haben und scheibenförmig sein. Die Frässcheibe verfügt über eine zentrale Bohrung zur Aufnahme eines Führungsstiftes, durch den die Frässcheibe am Knochen positioniert wird. Ein Durchgangsschlitz ermöglicht es, den Führungsstab mittels seitlicher Bewegung der Frässcheibe in der zentralen Bohrung zu platzieren. Eine Verbindung zwischen Frässcheibe und Schaft erfolgt über ein Schraubgewinde, wobei der Schaft in ein Gewinde an der Innenseite der zentralen Bohrung der Frässcheibe eingeschraubt wird. DE 21 2006 000 089 U1 offenbart die in der Präambel des Anspruchs 1 definierten Merkmale. Es ist eine Aufgabe der vorliegenden Erfindung, ein chirurgisches Fräswerkzeug und/oder Komponenten hiervon vorzuschlagen, welches bzw. welche in wenigstens einem der nachfolgenden Belange gegenüber dem Stand der Technik verbessert ist bzw. sind, nämlich einer vereinfachten Handhabung, zuverlässigen Benutzbarkeit, und/oder einer langen Haltbarkeit.

Gemäß einem Aspekt der Erfindung wird ein chirurgisches Fräswerkzeug zur Abtragung von Knochen oder Knorpelgewebe gemäß Anspruch 1 vorgeschlagen, das einen Fräskopf umfasst. Dieser ist mit einem zentralen Halteelement versehen. Das zentrale Halteelement weist eine Durchgangsöffnung entlang der Drehachse des Fräskopfes auf. Ein Führungsstab ist in der Durchgangsöffnung drehbar lagerbar, um den Fräskopf zu positionieren. Ein Schaft ist mit dem zentralen Halteelement durch einen Bajonettverschluss verbunden.

Der Bajonettverschluss umfasst einen Bajonettschlitz und einen Bajonettstift. Statt eines Bajonettschlitzes kann generell auch eine entsprechende Nut, ein Spalt, oder ein anderer weiblicher Bajonettmechanismus vorgesehen sein. Statt eines Bajonettstiftes kann generell auch ein entsprechender Pin, Nippel, Bolzen, eine Nocke, oder ein anderer männlicher Bajonettmechanismus vorgesehen sein. Gemäß der Erfindung umfasst der Bajonettverschluss einen Bajonettschlitz am Fräskopf und einen Bajonettstift am Schaft.

Bei diesen oder anderen Ausführungsformen kann ein Querschlitz am Bajonettschlitz vorgesehen sein. Der Bajonettstift kann bei distaler Bewegung des Schaftes in den Querschlitz verrastbar sein. Bei einer Ausführungsform, bei der Bajonettschlitz und Querschlitz am Fräskopf vorgesehen sind, kann sich der Querschlitz insbesondere ganz oder teilweise, vom Bajonettschlitz ausgehend, in distaler Richtung, d.h. vom Knochen weg bzw. zum Schaft hin erstrecken.

Mit der Erfindung wird ein modular aufgebautes Fräswerkzeug bereitgestellt, bei dem eine Kopplung zwischen Fräskopf und Schaft auf besonders einfache Weise erfolgt. So kann bspw. eine Schraubverbindung schwierig herstellbar sein, wenn sich der Fräskopf bereits am Knochen befindet und der Schaft in seiner Bewegung durch den Führungsstab behindert wird, der im praktischen Einsatz z.B. verbogen sein kann. Der Bajonettverschluss ermöglicht auch eine Führung des Fräskopfes in distaler Richtung (vom Körper bzw. Knochen weg), so dass ein Abheben des Fräskopfes möglich ist, um etwa während des Fräsvorganges die bisher erzielten Ergebnisse zu kontrollieren.

Ein Schraubgewinde kann sich beim Betrieb festsetzen, wobei sich die Kopplung etwa durch Zudrehen derart verfestigt, dass Schaft und Fräskopf nicht mehr einfach voneinander lösbar sind. Unter Umständen kann ein weiteres Werkzeug zum Lösen erforderlich sein, was zu komplexeren Abläufen und höheren Kosten führt.

Ein Festsetzen des Schaftes am Fräskopf kann alternativ oder zusätzlich auch auf Ansammlungen von Knochen- und/oder Weichteilfragmenten, Blut, Fett, usw. am Werkzeug zurückgehen. Der erfindungsgemäße Bajonettverschluss ist hier für ein Festsetzen allerdings weniger anfällig als andere Kopplungsarten einschließlich Schraubkopplungen. Darüber hinaus ist ein Bajonettverschluss auch leichter zu reinigen als etwa ein Schraubverschluss.

Der erfindungsgemäße Bajonettverschluss ist länger haltbar als andere Verschluss- bzw. Kopplungsarten. So kann etwa das Gewinde eines Schraubverschlusses vergleichsweise schnell unbrauchbar werden, etwa aufgrund von Abnutzung durch fehlgeschlagene Einschraubversuche, etc.

Ist der Bajonettschlitz am Fräskopf vorgesehen und der Bajonettstift am Schaft, so resultiert ein offener Aufbau des Fräskopfes, der damit im Vergleich zu Fräsköpfen mit Schraubgewinde etc. besonders leicht zu reinigen ist.

Ein am Bajonettschlitz vorgesehener Querschlitz kann der Aufrechterhaltung der Kopplung zwischen Fräskopf und Schaft dienen, also etwa der Sicherung gegen ein Zurückdrehen beim Abziehen des Fräskopfes, bspw. wenn der Chirurg während eines Fräsvorganges dessen Fortgang kontrolliert. Hierzu ist der Querschlitz ausgehend vom Bajonettschlitz vorzugsweise so vorgesehen, dass ein vom Bajonettschlitz entferntes Ende des Querschlitzes beim Zurückziehen des Schaftes dem Bajonettstift entgegensteht. Dies bedeutet, dass der Querschlitz sich in distaler Richtung vom Bajonettschlitz weg erstreckt, wenn der Bajonettstift am Schaft vorgesehen ist, und sich in proximaler Richtung vom Bajonettschlitz weg erstreckt, wenn der Bajonettstift am Fräskopf vorgesehen ist.

Bei manchen Ausführungsformen kann eine Wirkung, bspw. eine Federwirkung vorgesehen sein, um ein Verrasten des Bajonettstiftes im Querschlitz zu unterstützen. Bei einer Ausführungsform, bei der ein Bajonettschlitz und ein vom Bajonettschlitz in distaler Richtung wegführender Querschlitz am Fräskopf vorgesehen sind, kann bei verrastetem Bajonettstift vom Fräskopf auf den Schaft eine Federwirkung ausgeübt werden. Bspw. kann in einer Koppelhülse des Fräskopfes eine Feder vorgesehen sein. Allerdings sollte eine Ankopplung des Schaftes an den Fräskopf durch eine derartige Wirkung nicht wesentlich erschwert werden.

Der Querschlitz kann sich auch beidseitig vom Bajonettschlitz erstrecken (ohne oder mit gegenseitigem Versatz der beiden in distaler und proximaler Richtung abzweigenden Teil-Querschlitze), bspw. um neben einer Rückdrehsicherung auch eine besonders sichere und spielfreie Führung des Fräskopfes beim Fräsen bereitzustellen.

Statt nur eines Bajonettstiftes können auch mehrere Bajonettstifte mit einer entsprechenden Anzahl an Bajonettschlitzen verwendet werden. In einem Ausführungsbeispiel kann ein Schaft zwei, drei oder vier Bajonettstifte aufweisen, und der Fräskopf weist entsprechend zwei, drei, oder vier Bajonettschlitze auf. Mehrere Bajonettstifte bzw. -schlitze können bspw. verwendet werden, um ein Ankoppeln des Schaftes an den Fräskopf noch weiter zu vereinfachen, wenn sich bspw. mehrere Einkoppelmöglichkeiten ergeben. Auch kann sich eine Haltbarkeit von Fräsköpfen oder Schäften verlängern, und/oder ein Fräskopf kann offener gestaltet werden für eine leichtere Reinigung. Das Vorsehen nur eines Bajonettstiftes bzw. -schlitzes vereinfacht hingegen die Herstellung von Fräskopf bzw. Schaft und kann zu einer erleichterten Verbindung führen durch geringeren Widerstand beim Eindrehen des Bajonettstiftes in den Bajonettschlitz und/oder Querschlitz.

Das zentrale Halteelement weist einen Schlitz, genauer gesagt einen Führungsschlitz, zum Durchführen des Führungsstabs in die Durchgangsöffnung auf. Dies hat den großen Vorteil, dass der Fräskopf seitlich über den Führungsschlitz auf den am Knochen fixierten Führungsstab bis zum definitiven Sitz in der zentralen Durchgangsöffnung aufgeschoben werden kann. Somit wird der seitliche Platz zur Einführung des Fräskopfs über den Führungsstab minimiert.

Der Bajonettschlitz kann vom Führungsschlitz abzweigen. Ein separater Einführungsschlitz zum Einführen des Bajonettstiftes in den Bajonettschlitz kann dann entfallen, weil ein Teil des Führungsschlitzes hierfür verwendet wird. Herstellung und Wartung, z.B. Säuberung, des Fräskopfes vereinfachen sich entsprechend. Ein Querschlitz kann bspw. parallel zum Führungsschlitz verlaufen. Führungsschlitz, Bajonettschlitz und/oder Querschlitz können in einer Koppelhülse zur Kopplung von Fräskopf und Schaft ausgebildet sein.

Ein Führungsschlitz kann mit einer Phase versehen sein, um einen Durchgang des Führungsstabes zu erleichtern, bspw. auch wenn dieser verbogen ist. Eine Koppelhülse eines Fräskopfes kann mit einer Phase versehen sein, um ein Einführen eines Schaftes zu erleichtern. Ein Bajonettschlitz kann an einem Einkopplungsende eine Phase haben, um ein Einfangen und Einführen des Bajonettstiftes zu erleichtern. Der Bajonettschlitz kann sich vom Inneren eines Fräskopfes nach außen verjüngen, etwa beim Durchgang durch eine Wandung einer Koppelhülse. Dies erleichtert bspw. das Wegführen von Blut, Fett, etc. aus dem Bajonettschlitz. Gleiches gilt für einen Querschlitz. Sind Bajonettschlitz und ggf. Querschlitz statt am Fräskopf am Schaft vorgesehen, können sich auch hier Bajonettschlitz und/oder Querschlitz nach außen verjüngen.

Von Vorteil ist es darüber hinaus, wenn der Fräskopf mindestens ein Sichtfenster aufweist und der Führungsschlitz sich von einem Sichtfenster bis in die Durchgangsöffnung erstreckt. Sichtfenster ermöglichen den direkten Blick auf den bearbeiteten Knochenbereich und erstrecken sich beispielsweise vom Umfang des Fräskopfs radial nach innen, sodass die Länge des Führungsschlitzes von einem Sichtfenster durch das zentrale Halteelement in die Durchgangsöffnung reduziert ist und der Fräskopf an Stabilität gewinnt.

Es ist weiterhin von Vorteil, wenn der äußere Rand eine Breite zwischen 1 mm und 5 mm, bevorzugt zwischen 1 mm und 3 mm, aufweist. Eine solche Breite des äußeren Rands, der üblicherweise aus Instrumentenstahl gefertigt ist, garantiert eine ausreichende Stabilität des Fräskopfes und reduziert den zur Positionierung des Fräswerkzeugs benötigten freiliegenden Raum auf diese Breite.

Der Fräskopf weist vorteilhafterweise einen äußeren Rand sowie Schneidelemente auf. Die Schneidelemente sind so zwischen dem äußeren Rand und dem zentralen Halteelement angeordnet, dass mindestens ein Sichtfenster zwischen dem umfänglichen Rand und dem zentralen Halteelement ausgebildet ist. Der äußere Rand erhöht einerseits die Stabilität des Fräskopfs. Andererseits kann bereits bei einer seitlich freiliegenden Fläche, die lediglich der Breite des äußeren Rands entspricht, der Fräskopf senkrecht zur Fräsfläche eingesetzt werden und dann seitlich in den Gelenkspalt geschoben werden.

Dass der Schaft mit dem zentralen Halteelement durch einen Bajonettverschluss verbunden ist, hat u.a. den Vorteil, dass der Schaft durch eine einfache Bewegung radial kraft- und formschlüssig mit dem Fräskopf verbunden ist und somit die Drehbewegung des Schafts auf den Fräskopf übertragen wird. Des Weiteren kann der Fräskopf auch in Richtung der Drehachse vom Knochen axial abgehoben werden. Damit kann das Abtragen von Knochen einfach und insbesondere schnell unterbrochen sowie begutachtet werden.

Der Fräskopf kann scheibenförmig ausgeführt sein, bspw. mit einer flachen distalen Seite und einer flachen oder nur leicht gewölbten proximalen Seite. Die Herstellung einer gewünschten Kontur im Knochen kann so erleichtert werden. Vorteilhaft ist es ebenfalls, wenn der Fräskopf senkrecht zur Drehachse eine kreisförmige Außenkontur aufweist. Ist der Fräskopf in den Gelenkspalt eingeführt, so ist durch die kreisförmige Kontur sichergestellt, dass bei Rotation des Fräskopfes keine unerwarteten Hindernisse im gesamten Fräsradius auftreten.

Es ist ebenso von Vorteil, wenn der äußere Rand eine geschlossene Außenkontur aufweist. Dies verhindert ein Einhaken in Haltewerkzeuge oder auch Weichteile des umliegenden menschlichen Körpers und reduziert somit eine Verletzungsgefahr für den Patienten sowie Beschädigungen von sonstigen chirurgischen Werkzeugen und Störungen im Fräsablauf.

Gemäß einem weiteren Aspekt der Erfindung wird ein Fräskopf für ein chirurgisches Fräswerkzeug zur Abtragung von Knochen oder Knorpelgewebe gemäß Anspruch 8 vorgeschlagen. Der Fräskopf besitzt ein zentrales Halteelement, wobei das zentrale Halteelement eine Durchgangsöffnung entlang der Drehachse des Fräskopfes aufweist, in der ein Führungsstab drehbar lagerbar ist, um den Fräskopf zu positionieren. Ein Schaft ist mit dem zentralen Halteelement durch einen Bajonettverschluss verbindbar.

Ein Werkzeugsatz zum chirurgischen Fräsen kann eine Mehrzahl an Fräsköpfen nach dem vorangehenden Aspekt sowie einen Schaft umfassen. Die Fräsköpfe können sich bspw. nach Größe unterscheiden, so dass Gelenke oder sonstige Knochenstrukturen unterschiedlicher Größe bearbeitet werden können. Zusätzlich oder alternativ kann der Satz Fräsköpfe für unterschiedliche Anwendungsbereiche enthalten, also bspw. für Schultergelenke und für Hüftgelenke. Ein- und derselbe Schaft kann für die unterschiedlichen Köpfe vorgesehen sein, oder der Werkzeugsatz enthält zwei oder mehr Schäfte, bspw. für unterschiedliche Antriebsmechanismen. Der oder jeder Schaft ist mit einem zentralen Halteelement eines Fräskopfes oder mehrerer Fräsköpfe durch einen Bajonettverschluss verbindbar.

Die oben skizzierten Werkzeugsätze verwirklichen Vorteile, die mit modular aufgebauten Fräswerkzeugen verbunden sind; sind für einen Schaft mehrere Fräsköpfe vorgesehen, können bspw. Kosten für einen gegebenen Satz an Fräsköpfen gesenkt werden, bzw. zu gleichen Kosten kann eine größere Anzahl an Fräsköpfen bereitgestellt und dadurch eine bessere Anpassbarkeit an Gegebenheiten bei den Patienten erreicht werden; solche Gegebenheiten können etwa Gelenkgrößen betreffen.

Erfindungsgemäße Ausführungsbeispiele sind in den Zeichnungen beispielhaft dargestellt und werden anhand der nachfolgenden Beschreibung näher erläutert. Es zeigen:
- Fig. 1: ein Ausführungsbeispiel des erfindungsgemäßen Fräswerkzeugs, bei Anwendung an einer menschlichen Schulterpfanne (Glenoid) in perspektivischer Ansicht;
- Fig. 2: der Schaft des Fräswerkzeugs aus Fig. 1 in einer Seitenansicht;
- Fig. 3A: eine Detailansicht des Fräskopfs des Fräswerkwerkzeugs aus Fig. 1 in Draufsicht,
- Fig. 3B: eine Detailansicht des Fräskopfs des Fräswerkwerkzeugs aus Fig. 1 in Seitenansicht;
- Fig. 4: ein zweites Ausführungsbeispiel eines erfindungsgemäßen Fräskopfs mit abgeflachter Außenkontur in Draufsicht auf die Schneidfläche;
- Fig. 5: ein drittes Ausführungsbeispiel eines erfindungsgemäßen Fräskopfs ohne Sichtfenster in Draufsicht auf die Koppelhülse;
- Fig. 6A: ein erfindungsgemäßes Ausführungsbeispiel eines Fräskopfs beim seitlichen Einführen auf einen Führungsstab in schematischer Darstellung in einem ersten und
- Fig. 6B: ein erfindungsgemäßes Ausführungsbeispiel eines Fräskopfs beim Zentrieren auf einen Führungsstab in schematischer Darstellung in einem zweiten Schritt.
- Fig. 7A: eine Seitenansicht des Fräskopfes mit Bajonettschlitz der Fig. 3A, 3B;
- Fig. 7B: eine Schnittansicht des Fräskopfes mit Bajonettschlitz der Fig. 3A, 3B;
- Fig. 8A: einen Längsschnitt durch einen Rohling zur Herstellung des Schaftes aus Fig. 2;
- Fig. 8B: einen Stiftkörper zur Passung in den Rohling aus Fig. 8A; und
- Fig. 8C: einen Querschnitt durch den Rohling aus Fig. 8A mit eingepasstem Stiftköper aus Fig. 8B.

Einander entsprechende Teile sind in allen Figuren mit den gleichen Bezugszeichen versehen.

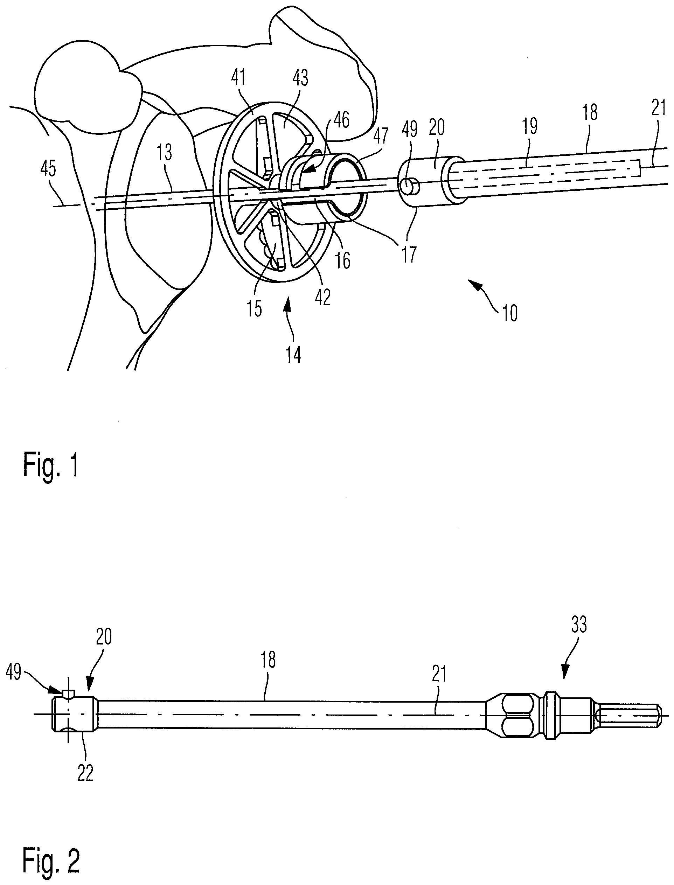

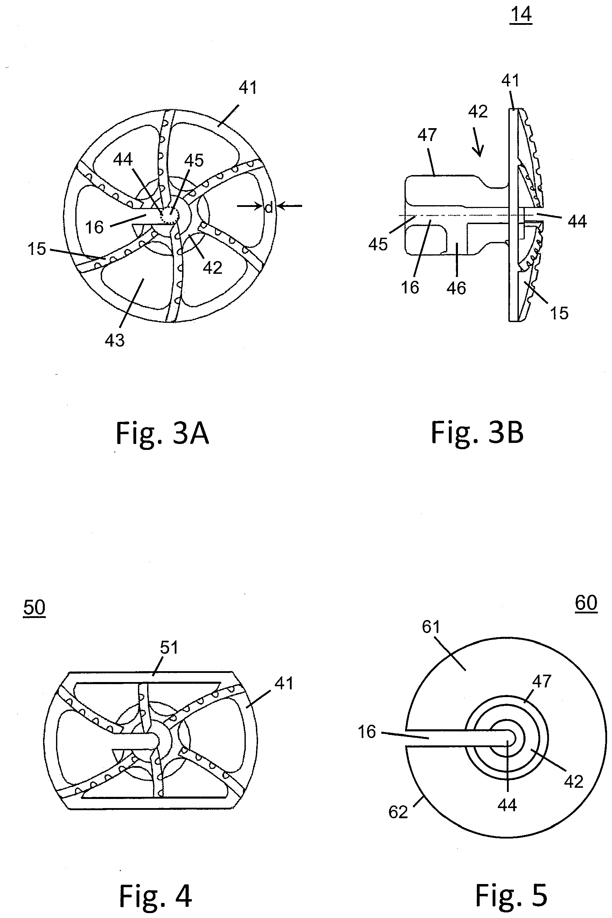

Fig. 1 zeigt die Positionierung eines modularen, chirurgischen Fräswerkzeugs 10 an einem linken menschlichen Schulterblatt 11, auch als Scapula bezeichnet, um eine Oberfläche einer Schulterpfanne 12, auch Cavitas Glenoidalis oder kurz Glenoid genannt, auszufräsen. Ein Führungsstab 13 ist starr in die Schulterpfanne 12 eingebracht und markiert das Zentrum der gewünschten rotationssymmetrischen Ausfräsung. Der Fräskopf 14 weist eine zentrale Durchgangsöffnung 44, siehe Fig. 3A, entlang der Drehachse 45 des Fräskopfs 14 auf. Der Fräskopf 14 wird bis zur zentralen Durchgangsöffnung 44 auf den Führungsstab 13 eingebracht und der Fräskopf 14 somit an der Schulterpfanne 12 positioniert.

In einer realen Operationssituation ist die Schulterpfanne 12 lediglich teilweise freigelegt, da Weichteile wie Muskeln und Bänder, hier zur besseren Übersicht nicht dargestellt, nur teilweise durch Haltewerkzeuge aus dem Operationsfeld weggezogen werden können. Dadurch kann ein rotationssymmetrischer Fräskopf 14 nicht in allen Fällen senkrecht zur Drehachse 45 über den Führungsstab 13 auf die Schulterpfanne 12 aufgesetzt werden. Der Fräskopf 14 weist jedoch mindestens ein Sichtfenster 43 zwischen einem äußeren Rand 41 und einem zentralen Halteelement 42 auf. Des Weiteren weist das zentrale Halteelement 42 einen Führungsschlitz 16 auf, der sich in radialer Richtung von einem Sichtfenster 43 zur zentralen Durchgangsöffnung 44 und in axialer Richtung durch das gesamte zentrale Halteelement 42 hindurch erstreckt. Der Fräskopf 14 wird somit durch Einführen auf den Führungsstab 13 durch ein Sichtfenster 43 und weiter über den Führungsschlitz 16 im Halteelement 42 in die zentrale Durchgangsöffnung 44 seitlich eingeschoben.

Ein Schaft 18 ist stabförmig ausgebildet und weist eine zentrale Ausnehmung 19 um die Längsachse 21 des Schafts 18 auf. Diese Ausnehmung 19 ist beispielsweise als Sackloch an dem dem Fräskopf 14 zugewandten Ende, wie in Fig. 1 dargestellt, oder als Durchgangsöffnung durch die gesamte Schaftlänge ausgeführt. Die Ausnehmung 19 weist einen Durchmesser auf, der in etwa dem Durchmesser des Führungsstabs 13 entspricht und eine Rotation des Schafts 18 um den unbeweglich fixierten Führungsstab erlaubt. Der Schaft 18 weist an dem dem Fräskopf 14 zugewandten Ende ein Koppelelement 20 zum Verbinden mit dem Fräskopf 14 auf.
Der Schaft 18 wird mit der zentralen Ausnehmung 19 über den Führungsstab 13 geschoben und das Koppelelement 20 mit einer Koppelhülse 47 eines zentralen Halteelements 42 des Fräskopfs 14 durch einen Bajonettverschluss 17 verbunden. Eine Rotation des Schafts 18 um seine Längsachse 21 wird somit auf den Fräskopf 14 übertragen. Die Position des Fräskopfs 14 zur Schulterpfanne 12 bleibt durch die Führung sowohl des Fräskopfs 14 als auch des Schafts 18 am Führungsstab 13 erhalten. Eine laterale Verschiebung wird verhindert. Der Bajonettverschluss ermöglicht zudem ein Abheben des Fräskopfes 14 vom zu bearbeitenden Knochen 12 in axialer Richtung nach Beendigung der Bearbeitung oder für Zwischenkontrollen.

Fig. 2 zeigt in einer Seitenansicht den Schaft 18 aus Fig. 1 vollständig, d.h. mit dem proximalen Koppelelement 20 zur Ankopplung an den Fräskopf 14 und einem lateralen Koppelelement 33 zur Ankopplung an einen mechanischen Antrieb (nicht gezeigt).

Das Koppelelement 20 des Schafts 18 umfasst ein Kopfstück 22 sowie einen Bajonettstift 49. Das Kopfstück 22 ist zur formschlüssigen Aufnahme in die Koppelhülse 47 des Fräskopfes 14 ausgebildet. Der Bajonettstift 49 ist korrespondierend zur Schlitzweite des Führungsschlitzes 16 (Fig. 1) sowie zur Schlitzweite des Bajonettschlitzes 46 ausgebildet, der vom Führungsschlitz 16 abzweigt und zusammen mit dem Bajonettstift 49 den Bajonettverschluss 17 bildet.

Wie aus Fig. 1 ersichtlich, wird der Schaft 18 über den Führungsstab 13 geschoben und mit der Kopplung 20 in die Koppelhülse 47 eingeführt. Der Bajonettstift 49 wird dabei durch den Führungsschlitz 16 bewegt. Durch Drehung des Schaftes 18 gelangt der Bajonettstift 49 in den Bajonettschlitz 46. Somit ergibt sich auf einfache Weise eine zuverlässige Verbindung zwischen Schaft 18 und Fräskopf 14, die sowohl eine Übertragung einer Rotationsbewegung des Schafts 18 auf den Fräskopf 14 ermöglicht, als auch ein vorübergehendes oder endgültiges Abheben des Fräskopfes 14 vom Knochen 11, 12.

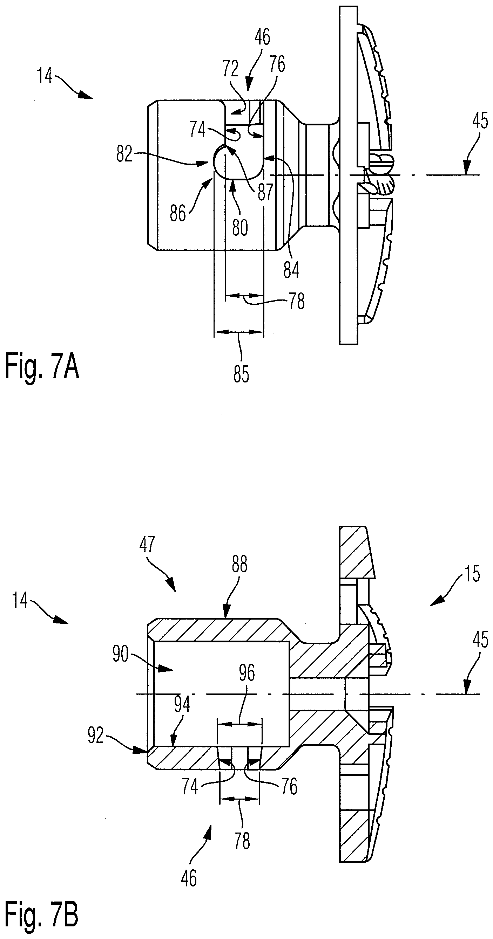

Der Bajonettschlitz 46 sowie das Zusammenspiel von Bajonettschlitz 46 und Bajonettstift 49 werden weiter unten anhand der Fig. 3B, 7A und 7B genauer beschrieben.

Neben einer starren Verbindung zwischen Schaft 18 und Fräskopf 14 ist auch ein kippbarer Koppelmechanismus, z.B. ein kippbarer Kardanmechanismus, möglich.

In Fig. 3A ist der Fräskopf 14 vergrößert in Draufsicht auf die dem Knochen zugewandte Seite dargestellt. Der Fräskopf 14 wird durch ein zentrales Halteelement 42 und einen äußeren Rand 41 gebildet, die konzentrisch um eine Drehachse 45 angeordnet sind. Zwischen dem zentralen Halteelement 42 und dem äußeren Rand 41 erstrecken sich mehrere Schneidelemente 15, die das zentrale Halteelement 42 und den äußeren Rand 41 miteinander verbinden. Ein Schneidelement 15 kann aus einem Träger und einer oder mehreren aufgesetzten Schneiden oder einstückig ausgebildet sein. Dabei bleibt mindestens ein Sichtfenster 43, das durch zwei Schneidelemente 43, den äußeren Rand 41, sowie dem zentralen Halteelement 42 begrenzt ist, frei und bildet eine durchgängige Ausnehmung. Die Ausdehnung des Sichtfensters 43 entspricht mindestens dem Durchmesser des Führungsstabs, sodass der Führungsstab 13 hindurchgeführt werden kann. Die Sichtfenster 43 dienen außerdem zum Abführen von abgetragenen Knochenspänen und andererseits gewähren sie eine direkte Sicht auf den abzutragenden Knochen. Im dargestellten Ausführungsbeispiel erstrecken sich die Schneidelemente 15 beispielsweise Speichen-förmig radial vom zentralen Halteelement 42 zum äußeren Rand 41 und bilden mehrere gleichförmige Sichtfenster 43.

Der äußere Rand 41 weist einen Durchmesser von 1 - 5 mm bevorzugt zwischen 1 mm und 3 mm auf. Der äußere Rand 41 hat eine geschlossene Außenkontur. Dadurch wird ein Anstoßen oder Einhaken des Fräskopfs 14 in beispielsweise Knochenhaken zum Abhalten von Weichteilen verhindert.

Entlang der Drehachse 45 des Fräskopfs 14 durch das zentrale Halteelement 42 hindurch ist die zentrale Durchgangsöffnung 44, hier gestrichelt gezeichnet, angeordnet. Ein Führungsschlitz 16 erstreckt sich von einem Sichtfenster 43 bis in die zentrale Durchgangsöffnung 44 und erlaubt somit das seitliche Einführen des Fräskopfes 14 über den Führungsstab 13 in die zentrale Durchgangsöffnung 44. Der Führungsstab 13 ist dabei beispielsweise parallel zur Drehachse 45 des Fräskopfes 14 ausgerichtet. Der Führungsschlitz 16 kann, wie dargestellt, die gleiche Weite wie die zentrale Durchgangsöffnung 44 aufweisen. Der Schlitz kann aber auch nach radial außen hin aufgeweitet geformt sein.

Fig. 3B zeigt den Fräskopf 14 in einer Seitenansicht. Die Schneidelemente 15 bilden eine konvex geformte Kontur. Andere, z.B. flache oder konkave Konturen sind je nach Form der gewünschten Ausnehmung im Knochen möglich. Um jedoch bei extrem geringem Operationsplatz das Positionieren des Fräskopfs zu ermöglichen, ist auch ein geschlitzter äußerer Rand 41, mit einem Schlitz, der in das Sichtfenster 43 führt, das mit dem seitlichen Führungsschlitz 16 im zentralen Halteelement 42 mit der zentralen Durchgangsöffnung 44 verbunden ist, möglich.

Das zentrale Halteelement 42 ist mit den Schneidelementen 15 verbunden bzw. kann auch einstückig mit den Schneidelementen 15 ausgebildet sein. Das zentrale Halteelement 42 umfasst eine Koppelhülse 47, die zur Kopplung mit dem Schaft 18 dient. Das zentrale Halteelement 42 und die Koppelhülse 47 können monolithisch als ein Element oder mehrteilig miteinander verbunden ausgeführt sein. In der Koppelhülse 47 geht die um die Drehachse 45 angeordnete zentrale Durchgangsöffnung 44 in eine Form über, die die Aufnahme des Schafts 18 ermöglicht. Die Koppelhülse 47 weist in axialer Richtung den Führungsschlitz 16 auf, von dem eine weitere Ausnehmung bzw. ein Bajonettschlitz 46 in radialer Richtung für den Bajonettverschluss 17 abzweigt. In die Ausnehmung 46 ist der in Fig. 1 gezeigte und anhand von Fig. 2 genauer beschriebene Nippel bzw. Bajonettstift 49 einführbar.

Fig. 4 zeigt einen Fräskopf 50 mit geschlossener, aber abgeflachter Außenkontur 51, bei der zwei gegenüberliegende Seiten des äußeren Rands 41 eben ausgebildet sind. Der erfindungsgemäße Fräskopf kann auch eine ovale Außenkontur aufweisen.

Der Fräskopf 14, 50 sowie der Schaft 18 sind beispielsweise aus Instrumentenstahl hergestellt. Der zur Zentrierung verwendete Führungsstab, beispielsweise ein Kirschner-Draht, hat einen Durchmesser von beispielsweise 1 - 4 mm, bevorzugt von 1 - 2 mm. Die zentrale Durchgangsöffnung 44 weist den gleichen oder einen etwas größeren Durchmesser auf.

Fig. 5 zeigt ein weiteres Ausführungsbeispiel eines erfindungsgemäßen Fräskopfs 60 in Draufsicht in Blickrichtung auf die Koppelhülse 47. Der Fräskopf 60 weist eine geschlossene Frässcheibe 61 ohne Sichtfenster auf. Der Führungsschlitz 16 erstreckt sich radial von der zentralen Durchgangsöffnung 44 des zentralen Halteelements 42 bis zum umfänglichen Rand 62 der Frässcheibe 61 und in axialer Richtung durch das gesamte zentrale Halteelement 42 und die Frässcheibe 61 hindurch. Die Frässcheibe 61 hat beispielsweise eine runde Außenkontur. Die Außenkontur kann aber auch abgeflacht, oval oder anders ausgeformt sein. Ein Fräskopf 60 mit einer Frässcheibe 61 weist eine hohe Stabilität auf, erlaubt eine flexible Gestaltung der Schneiden und ist kostengünstiger herstellbar.

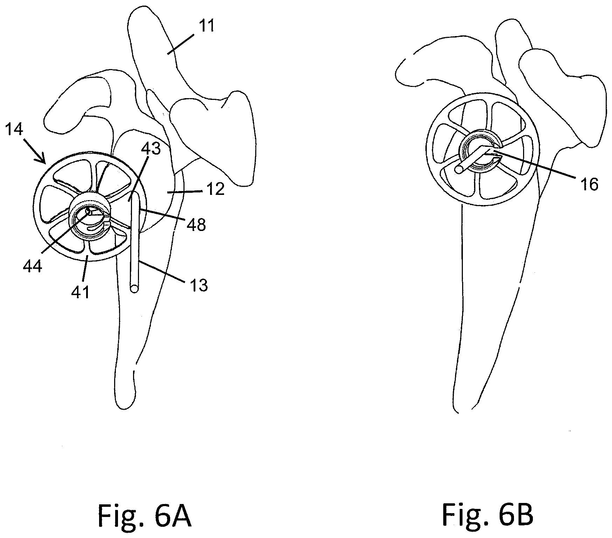

Die Figuren 6A und 6B zeigen in zwei Schritten das Einführen des Fräskopfs 14 in den Führungsstab 13, der das Zentrum der Ausfräsung in einem Schulterblatt 11 bzw. der Schulterpfanne 12 markiert. In einem realen Operationsszenario sind weite Teile des Schulterblatts und der dargestellten Schulterpfanne 12 durch Weichteile verdeckt. Der rechts des Führungsstabs 13 liegende Teil der Schulterpfanne 12 ist oftmals nur durch einen kleinen Spalt zugänglich. Der Führungsstab 13 wird nun über das Sichtfenster 43 in den Fräskopf 14 eingeführt. Der Fräskopf 14 wird mit der Innenseite 48 des äußeren Rands 41 entlang des Führungsstabs 13 in Richtung Knochen geführt. Ist der Fräskopf 14 auf der Höhe des Gelenkspalts angelangt, wird der Fräskopf 14 seitlich nach rechts geschoben und dabei der Führungsstab 13 in den Führungsschlitz 16 eingeschoben, bis dieser in der zentralen Durchgangsöffnung 44 liegt.

Die Endposition ist in Fig. 6B gezeigt. Durch das Einführen des Führungsstabs 13 in die zentrale Ausnehmung 19 des Schafts 18, wird der Fräskopf 14 in der gewünschten Lage positioniert und fixiert. Durch die Verbindung des Koppelelements 20 des Schafts 18 mit der Koppelhülse 47 des zentralen Halteelements 42 werden Schaft 18 und Fräskopf 14 lösbar miteinander verbunden.

Das Einführen des Führungsstabs 13 in den Fräskopf 14 sowie das Einführen des Führungsstabs 13 in die zentrale Ausnehmung 19 des Schafts 18 sind dabei als relative Bewegung zwischen Führungsstab 13 und Fräskopf 14 bzw. Führungsstab 13 und Schaft 18 zu verstehen. In allen Fällen bleibt der Führungsstab 13 ortsfest und der Fräskopf 14 und der Schaft 18 werden bewegt.

Fig. 7A zeigt den Fräskopf 14 in einer Seitenansicht vergleichbar zu derjenigen der Fig. 3B, jedoch um etwa 90° um die Drehachse 45 gedreht, so dass weitere Eigenschaften des Bajonettschlitzes 46 erkennbar werden. Der Bajonettschlitz 46 zweigt vom Führungsschlitz 16 ab, von dem in der Fig. 7A nur noch die dem Abzweig gegenüberliegende Wandung 72 erkennbar ist. Den Bajonettschlitz 46 in lateraler und proximaler Richtung begrenzende Wandungen 74 und 76 definieren eine Schlitzweite 78, die zu einem Durchmesser des Bajonettstiftes am Schaft korrespondiert.

Der Bajonettschlitz 46 verläuft vom Führungsschlitz 16 bis zu einem entgegengesetzten Ende 80, wo sich ein Querschlitz 82 befindet. Dieser erstreckt sich im wesentlichen senkrecht zur Verlaufsrichtung des Bajonettschlitzes 46, d.h. entlang der Drehachse 45. Ein proximales Ende 84 des Querschlitzes 82 entspricht in dem hier gezeigten Ausführungsbeispiel der Wandung 76 des Bajonettschlitzes 46. Ein laterales Ende 86 des Querschlitzes 82 ist jedoch in lateraler Richtung gegenüber der Wandung 74 des Bajonettschlitzes 46 weiter zurückversetzt, so dass der Querschlitz 82 an seinem Ende 86 eine Ausnehmung zur Aufnahme eines Bajonettstiftes bildet, wenn dieser in lateraler Richtung bewegt wird.

Die Fig. 7B zeigt einen Querschnitt durch den Fräskopf 14 mit Schneidelementen 15 und Koppelhülse 47. Letztere bildet mit ihrer Wandung 88 eine zur Aufnahme eines Schaftes bestimmte Öffnung 90. Die Wandung 88 ist am lateralen Ende der Koppelhülse 47 mit einer Phase 92 ausgebildet. Dies kann die Aufnahme des Schaftes erleichtern, bspw. wenn der Führungsdraht oder -stab (Bezugsziffer 13 in Fig. 1, 6A) verbogen ist.

In der Schnittansicht der Fig. 7B ist auch der Bajonettschlitz 46 erkennbar. Dessen Wandungen 74 und 76 haben eine von 90° abweichende Neigung gegenüber der Drehachse 45 und definieren daher an der Außenseite der Koppelhülse 47 die bereits in Fig. 7A angedeutete Schlitzweite 78, an der inneren Wandung 94 der Schaftaufnahmeöffnung 90 jedoch eine größere Schlitzweite 96. Die konusartige Erweiterung des Bajonettschlitzes 46 nach innen ermöglicht u.a. eine erleichterte Abführung von Blut, Fett, Gewebe- oder Knochenfragmenten, die durch den Bajonettschlitz 46 oder Querschlitz 82 eintreten. Auf diese Weise wird die Bewegung des Bajonettstiftes im Bajonettschlitz 46 erleichtert. Auch der Querschlitz 82 kann mit einer entsprechenden konusartigen Erweiterung von außen nach innen (bzw. Verjüngung von innen nach außen) ausgeführt sein, wodurch sich auch hier die entsprechenden Vorteile ergeben.

Anhand der Fig. 1 und 2 wurde bereits ein Zusammenwirken von Schaft 18 und Fräskopf 14 beschrieben; dies wird hier unter zusätzlicher Bezugnahme insbesondere auf Fig. 7A fortgeführt. Zur Herstellung einer Verbindung zwischen Schaft 18 und Kopf 14 wird der Bajonettstift 49 von Schaft 18 mittels translatorischer Bewegung des Schaftes 18 in proximaler Richtung in den Führungsschlitz 16 eingeführt. Durch rotatorische Bewegung des Schaftes 18 wird der Bajonettstift 49 sodann in den Bajonettschlitz 46 geführt. Ein Fräsvorgang zum Abfräsen von Material vom Knochen 11, 12 wird durch weitere rotatorische Bewegung des Schaftes 18 in die gleiche Richtung bewirkt. Durch Umkehr der rotatorischen Bewegungsrichtung wird der Bajonettstift 49 durch den Bajonettschlitz 46 zurück zum Führungsschlitz 16 bewegt. Ein Lösen der Verbindung zwischen Schaft 18 und Fräskopf 14 kann dann durch translatorische Bewegung des Schaftes 18 in distaler Richtung erfolgen, wobei der Bajonettstift 49 durch den Führungsschlitz 16 zurückbewegt wird.

Sind Schaft 18 und Fräskopf 14 miteinander verbunden und befindet sich der Bajonettstift 49 insbesondere am Anschlag des Bajonettschlitzes 46, d.h. an dessen Ende 80 (Fig. 7A), so gerät der Bajonettstift 49 durch translatorische Bewegung des Schaftes 18 in distaler Richtung an das distale Ende bzw. den distalen Anschlag 86 des Querschlitzes 82. Fortgesetzte Bewegung des Schaftes 18 führt zu einem Abheben des Fräskopfes 14 vom Knochen 11, 12 (vgl. Fig. 1). Dabei ist der Schaft 18 im Fräskopf 14 verrastet, d.h. die über die Breite 78 des Bajonettschlitzes 46 hinausgehende Längserstreckung 85 des Querschlitzes bzw. die Nase 87 zwischen Bajonettschlitz 46 und Querschlitz 82 sichert den Schaft 18 im Fräskopf 14, so dass eine Rückbewegung des Bajonettstiftes 49 durch den Bajonettschlitz 46 und ungewolltes Lösen der Verbindung zwischen Schaft 18 und Fräskopf 14 verhindert wird. Ein Verlieren des Fräskopfes 14 beim Abziehen wird verhindert und somit wird ein vorübergehendes oder endgültiges Abheben des Fräskopfes 14 vom Knochen 11, 12 zuverlässig ermöglicht.

In dem in Fig. 7A gezeigten Ausführungsbeispiel zweigt der Bajonettschlitz 46 im Wesentlichen senkrecht vom Führungsschlitz 16 ab. Bei anderen Ausführungsbeispielen verläuft der Bajonettschlitz in einem anderen als einem rechten Winkel zum Bajonettschlitz, bspw. in einem Winkel von 135°, oder 45°, oder in einem spitzeren oder stumpferen Winkel.

Bei diesen oder anderen Ausführungsbeispielen kann der Querschlitz 82 eine größere Längserstreckung haben als in Fig. 7A gezeigt. In distaler Richtung ist allerdings ein Einrücken oder Einrasten des Bajonettstiftes 49 über die Nase 87 an den Anschlag 86 des Querschlitzes 82 ausreichend um ein zuverlässiges Abheben des Fräskopfes 14 zu gewährleisten, d.h. eine Länge des Querschlitzes von der doppelten Breite des Bajonettschlitzes (oder weniger) ist für ein zuverlässiges Verrasten beim Abheben ausreichend.

Der Querschlitz muss sich nicht an einem Ende des Bajonettschlitzes befinden, sondern kann an einer anderen Stelle vom Bajonettschlitz abzweigen. Dann ist vor einer translatorischen Bewegung zum Abheben des Fräskopfes ggf. noch eine entsprechende Rückbewegung des Schaftes erforderlich, um den Bajonettstift vom Anschlag des Bajonettschlitzes zum Querschlitz zu bewegen.

Der Querschlitz kann sich in proximaler Richtung näher zum Knochen erstrecken als der Bajonettschlitz. Es können statt nur einem Querschlitz auch zwei oder mehr Schlitze quer zum Bajonettschlitz vorgesehen sein. In Fig. 7A ist gezeigt, dass sich der Querschlitz im wesentlichen senkrecht zum Bajonettschlitz erstreckt. Bei anderen Ausführungsbeispielen verläuft der Querschlitz in einem anderen als einem rechten Winkel zum Bajonettschlitz, bspw. in einem Winkel von 135°, oder 45°, oder in einem spitzeren oder stumpferen Winkel.

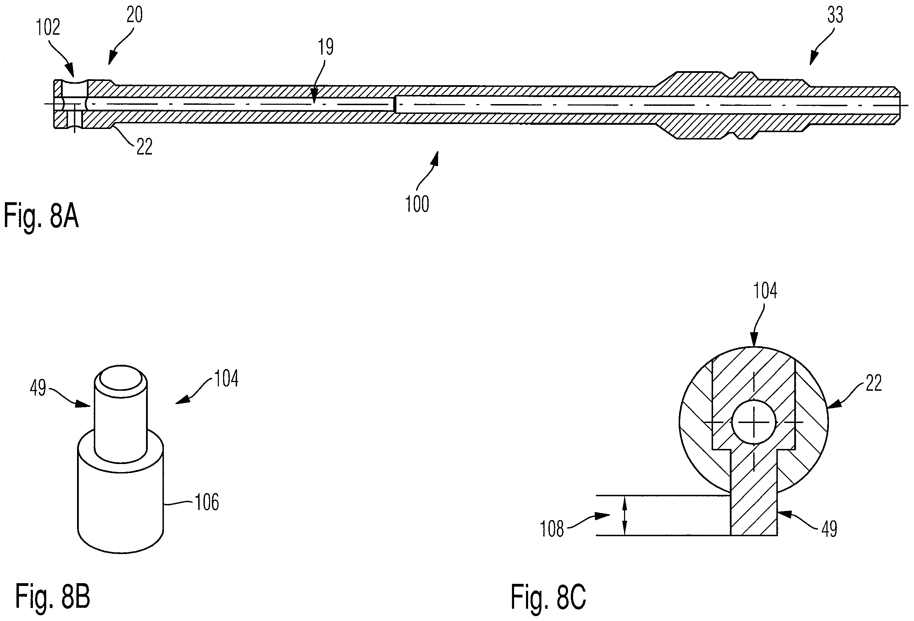

Die Fig. 8A bis 8C illustrieren ein Verfahren zur Herstellung des Schaftes 18 aus Fig. 2. Fig. 8A zeigt einen Querschnitt durch ein Werkstück 100, aus dem der Schaft 18 hergestellt wird. Das Werkstück 100 verfügt bereits über das Koppelelement 20 mit Kopfstück 22 sowie das Koppelelement 33. In einem Verfahrensschritt S1 wird eine Bohrung zur Herstellung der zentralen Ausnehmung 19 eingebracht. In einem Schritt S2, der parallel, vor- oder nachgelagert zum Schritt S1 sein kann, wird eine Bohrung 102 in das Kopfstück 22 eingebracht.

Fig. 8B zeigt einen Stiftkörper 104 mit Basis 106 und Stift 49. Der Stiftkörper 104 wird vorgelagert als separates Werkstück hergestellt. In einem Schritt S3 wird der Stiftkörper 104 in die Bohrung 102 im Rohling 100 eingepasst. Fig. 8C zeigt einen Schnitt durch das Koppelelement 20 des Werkstücks 100 mit eingepasstem Stiftkörper 104. Nach der Einpassung bildet der Stiftkörper 104 den Bajonettstift 49 aus, der um eine Stiftlänge 108 aus dem Kopfstück 22 heraussteht.

In einem Schritt S4 wird der Stiftkörper 104 in seiner Passung im Kopfstück 22 befestigt, bspw. mittels Laserschweissen. Der Schritt S4 kann eine Überarbeitung des Kopfstücks 22 umfassen, um etwa einen gratfreien Sitz des Stiftkörpers 104 sicherzustellen und/oder die Stiftlänge 108 des Bajonettstifts 49 an exakte Vorgaben anzupassen. Die Länge 108 des Bajonettstiftes 49 sollte ein sicheres Einfügen und eine sichere Verbindung des Schaftes 18 mit dem Fräskopf 14 erlauben, ohne dass der Stift 49 aus der Koppelhülse 47 wesentlich heraussteht. Der Stift 49 sollte nicht so weit aus der Koppelhülse 47 herausstehen, dass ein Einführen des Schaftes 18 in den Fräskopf 14, ein Fräsvorgang, ein Abheben des Fräskopfes 14 vom Knochen, und/oder ein Lösen des Schaftes 18 vom Fräskopf 14 behindert wird, bspw. weil der Stift 49 in Kontakt mit Körpergewebe kommt.

In einem Schritt S5 wird die Bohrung 19 durch den in das Kopfstück 22 eingepassten Stiftkörper 104 hindurch nachgefertigt. Damit ist das Herstellungsverfahren für den Schaft 18 beendet. Bei einem alternativen Herstellungsverfahren kann die Bohrung zur Aufnahme des Führungsstabes auch vollständig der Einpassung des Stiftkörpers nachgelagert werden, d.h. der Schritt S1 entfällt und wird zusammen mit dem Schritt S5 nach den Schritten S2 - S4 durchgeführt.

Die beschriebenen modularen chirurgischen Fräswerkzeuge sowie Komponenten hiervon können neben der Präparation von Schultergelenken auch zur Behandlung anderer menschlicher oder tierischer Körperbereiche angewendet werden, beim Menschen etwa zur Herstellung von Ausnehmungen an der Hüftpfanne oder für die Präparation des Oberarmkopfes auch auf der Oberarmseite.

Die Erfindung ist nicht auf die hier beschriebenen Ausführungsbeispiele und die darin hervorgehobenen Aspekte beschränkt; vielmehr sind innerhalb des durch die anhängenden Ansprüche angegebenen Bereichs eine Vielzahl von Abwandlungen möglich, die im Rahmen fachmännischen Handelns liegen. Dies schließt Kombinationen mehrerer Merkmale oder Merkmalsgruppen ein, auch wenn diese vorstehend unabhängig voneinander beschrieben sind.

## Patentansprüche

1. Chirurgisches Fräswerkzeug (10, 30) zur Abtragung von Knochen oder Knorpelgewebe,
mit einem Fräskopf (14, 50, 60) mit einem zentralen Halteelement (42), wobei das zentrale Halteelement (42) eine Durchgangsöffnung (44) entlang der Drehachse (45) des Fräskopfes (14) aufweist, in der ein Führungsstab (13) drehbar lagerbar ist, um den Fräskopf (14) zu positionieren,
wobei ein Schaft (18) mit dem zentralen Halteelement (42) durch einen Bajonettverschluss (17) verbunden ist, und wobei der Bajonettverschluss (17) einen Bajonettschlitz (46) am Fräskopf (14) und einen Bajonettstift (49) am Schaft (18) umfasst,
**dadurch gekennzeichnet,**
**dass** das zentrale Halteelement (42) einen Führungsschlitz (16) zum Durchführen des Führungsstabs (13) in die Durchgangsöffnung (44) aufweist.

2. Chirurgisches Fräswerkzeug nach Anspruch 1,
**dadurch gekennzeichnet,**
**dass** ein Querschlitz (82) am Bajonettschlitz (46) vorgesehen ist, wobei der Bajonettstift (49) bei distaler Bewegung des Schaftes (18) in den Querschlitz (82) verrastbar ist.

3. Chirurgisches Fräswerkzeug nach Anspruch 1,
**dadurch gekennzeichnet,**
**dass** der Bajonettschlitz (46) vom Führungsschlitz (16) abzweigt.

4. Chirurgisches Fräswerkzeug nach einem der vorhergehenden Ansprüche,
**dadurch gekennzeichnet,**
**dass** Bajonettschlitz (46) und/oder Querschlitz (82) sich nach außen verjüngen.

5. Chirurgisches Fräswerkzeug nach Anspruch 1,
**dadurch gekennzeichnet,**
**dass** der Fräskopf (14) mindestens ein Sichtfenster (43) aufweist und der Führungsschlitz (16) sich von einem Sichtfenster (43) bis in die Durchgangsöffnung (44) erstreckt.

6. Chirurgisches Fräswerkzeug nach einem der vorhergehenden Ansprüche,
**dadurch gekennzeichnet,**
**dass** ein äußerer Rand (41) des Fräskopfes (14) eine geschlossene Außenkontur bildet, wobei der äußere Rand (41) einen Schlitz zum Durchführen des Führungsstabs (13) aufweist.

7. Chirurgisches Fräswerkzeug nach einem der vorhergehenden Ansprüche,
**dadurch gekennzeichnet,**
**dass** der Schaft (18) eine um seine Längsachse (21) zentrierte Ausnehmung (19) zur Aufnahme des Führungsstabs (13) aufweist.

8. Fräskopf für ein chirurgisches Fräswerkzeug (10, 30) zur Abtragung von Knochen oder Knorpelgewebe,
mit einem zentralen Halteelement (42),
wobei das zentrale Halteelement (42) eine Durchgangsöffnung (44) entlang der Drehachse (45) des Fräskopfes (14) aufweist, in der ein Führungsstab (13) drehbar lagerbar ist, um den Fräskopf (14) zu positionieren,
wobei ein Schaft (18) mit dem zentralen Halteelement (42) durch einen Bajonettverschluss (17) verbindbar ist, und
wobei der Bajonettverschluss (17) einen Bajonettschlitz (46) am Fräskopf (14), und einen Bajonettstift (49) am Schaft (18) umfasst,
**dadurch gekennzeichnet,**
**dass** das zentrale Halteelement (42) einen Führungsschlitz (16) zum Durchführen des Führungsstabs (13) in die Durchgangsöffnung (44) aufweist.

9. Werkzeugsatz zum chirurgischen Fräsen, umfassend eine Mehrzahl an Fräsköpfen nach Anspruch 8 und einen Schaft, wobei die Fräsköpfe sich nach Größe und/oder vorgesehenem Anwendungsbereich am Körper unterscheiden und der Schaft (18) mit einem zentralen Halteelement (42) eines jeden Fräskopfes (14) durch einen Bajonettverschluss (17) verbindbar ist.

## Claims

1. Surgical milling tool (10, 30) for removing bone or cartilaginous tissue,
with a milling head (14, 50, 60) with a central holding element (42), wherein the central holding element (42) has a through-opening (44) along the rotational axis (45) of the milling head (14) in which a guide rod (13) can be mounted rotatably in order to position the milling head (14),
wherein a shank (18) is connected with the central holding element (42) by a bayonet connection (17), and wherein the bayonet connection (17) comprises a bayonet slot (46) on the milling head (14) and a bayonet pin (49) on the shank (18),
**characterised in that**
the central holding element (42) has a guide slot (16) for guiding the guide rod (13) through into the through-opening (44).

2. Surgical milling tool according to claim 1,
**characterised in that**
a transverse slot (82) is provided in the bayonet slot (46), wherein the bayonet pin (49) can be locked into the transverse slot (82) during distal movement of the shank (18).

3. Surgical milling tool according to claim 1,
**characterised in that**
the bayonet slot (46) branches off the guide slot (16).

4. Surgical milling tool according to one of the preceding claims,
**characterised in that**
the bayonet slot (46) and/or the transverse slot (82) taper outwards.

5. Surgical milling tool according to claim 1,
**characterised in that**
the milling head (14) has at least one viewing window (43) and the guide slot (16) extends from a viewing window (43) into the through-opening (44).

6. Surgical milling tool according to one of the preceding claims,
**characterised in that**
an outer edge (41) of the milling head (14) forms a closed outer contour, wherein the outer edge (41) has a slot for guiding the guide rod (13) through.

7. Surgical milling tool according to one of the preceding claims,
**characterised in that**
the shank (18) has a recess (19) which is centred about its longitudinal axis (21) for receiving the guide rod (13).

8. Milling head for a surgical milling tool (10, 30) for removing bone or cartilaginous tissue, with a central holding element (42),
wherein the central holding element (42) has a through-opening (44) along the rotational axis (45) of the milling head (14) in which a guide rod (13) can be mounted rotatably in order to position the milling head (14),
wherein a shank (18) can be connected with the central holding element (42) by a bayonet connection (17), and
wherein the bayonet connection (17) comprises a bayonet slot (46) on the milling head (14) and a bayonet pin (49) on the shank (18),
**characterised in that**
the central holding element (42) has a guide slot (16) for guiding the guide rod (13) through into the through-opening (44).

9. Tool kit for surgical milling, comprising a plurality of milling heads according to claim 8 and a shank, wherein the milling heads differ according to size and/or intended area of use on the body and the shank (18) can be connected with a central holding element (42) of each milling head (14) by a bayonet connection (17).

## Revendications

1. Outil de fraisage chirurgical (10,30) pour l'enlèvement d'os ou de cartilages,
avec une tête de fraisage (14, 50, 60) avec un élément de maintien central (42), l'élément de maintien central (42) comprenant une ouverture de passage (44) le long de l'axe de rotation (45) de la tête de fraisage (14), dans laquelle une tige de guidage (13) peut être logée de manière rotative, afin de positionner la tête de fraisage (14),
une tige (18) étant reliée avec l'élément de maintien central (42) par l'intermédiaire d'un raccordement à baïonnette (17) et le raccordement à baïonnette (17) comprenant une fente à baïonnette (46) au niveau de la tête de fraisage (14) et une tige de baïonnette (49) au niveau de la tige (18),
**caractérisé en ce que**
l'élément de maintien central (42) comprend une fente de guidage (16) pour le passage de la tige de guidage (13) dans l'ouverture de passage (44).

2. Outil de fraisage chirurgical selon la revendication 1, **caractérisé en ce qu'**une fente transversale (82) est prévue sur la fente à baïonnette (46), la tige de baïonnette (49) pouvant être encliquetée dans la fente transversale (82) lors d'un mouvement distal de la tige (18).

3. Outil de fraisage chirurgical selon la revendication 1, **caractérisé en ce que** la fente à baïonnette (46) diverge de la fente de guidage (16).

4. Outil de fraisage chirurgical selon l'une des revendications précédentes, **caractérisé en ce qu'**une fente à baïonnette (46) et/ou la fente transversale (82) se rétrécissent vers l'extérieur.

5. Outil de fraisage chirurgical selon la revendication 1, **caractérisé en ce que** la tête de fraisage (14) comprend au moins une fenêtre de visualisation (43) et la fente de guidage (16) s'étend d'une fenêtre de visualisation (43) jusqu'à l'ouverture de passage (44).

6. Outil de fraisage chirurgical selon l'une des revendications précédentes, **caractérisé en ce qu'**un bord extérieur (41) de la tête de fraisage (14) forme un contour externe fermé, le bord extérieur (41) comprenant une fente pour le passage de la tige de guidage (13).

7. Outil de fraisage chirurgical selon l'une des revendications précédentes, **caractérisé en ce que** la tige (18) comprend un évidement (19) centré autour de son axe longitudinal (21) pour le logement de la tige de guidage (13).

8. Tête de fraisage pour un outil de fraisage chirurgical (10, 30) pour l'enlèvement d'os ou de cartilages, avec un élément de maintien central (42), l'élément de maintien central (42) comprenant une ouverture de passage (44) le long de l'axe de rotation (45) de la tête de fraisage (14), dans laquelle une tige de guidage (13) peut être logée de manière rotative, afin de positionner la tête de fraisage (14),
une tige (18) pouvant être reliée avec l'élément de maintien central (42) par l'intermédiaire d'un raccordement à baïonnette (17) et
le raccordement à baïonnette (17) comprenant une fente à baïonnette (46) sur la tête de fraisage (14) et comprenant une tige de baïonnette (49) sur la tige (18),
**caractérisé en ce que**
l'élément de maintien central (42) comprenant une fente de guidage (16) pour le passage de la tige de guidage (13) dans l'ouverture de passage (44).

9. Ensembles d'outils pour le fraisage chirurgical comprenant une pluralité de têtes de fraisage selon la revendication 8 et une tige, les têtes de fraisage se distinguant par leur taille et/ou leur domaine d'application prévu sur le corps et la tige (18) pouvant être reliée avec un élément de maintien central (42) de chaque tête de fraisage (14) par l'intermédiaire d'un raccordement à baïonnette (17).
